# EUROPÄISCHE PATENTANMELDUNG

(11) **EP 1 925 673 A1**
(43) Veröffentlichungstag der Anmeldung: **28.05.2008**
(21) Anmeldenummer: 06023997.7
(22) Anmeldetag: 20.11.2006
(51) Int. Cl.: C12P 7/64, C07C 59/44, C07C 51/41

(54) **Verfahren zur Herstellung von Zinkrizinoleat**

(71) Anmelder: Cognis Oleochemicals GmbH, 40589 Düsseldorf (DE)
(72) Erfinder: Schörken, Ulrich Dr., 40591 Düsseldorf (DE); Both, Sabine Dr., 41470 Neuss (DE); Stuhlmann, Diana, 40627 Düsseldorf (DE)
(74) Vertreter: Reinhardt, Jürgen

(57) **Zusammenfassung**

Beschrieben wird ein einstufiges Verfahren zur Herstellung Zinkricinoleat, wobei Ricinusöl in Gegenwart geeigneter Enzyme, vorzugsweise von Lipasen gespalten und gleichzeitig mit Zinkoxid in wässriger Lösung zum Zinkricinoleat umgesetzt wird. Das so hergestellte Produkt weist einen extrem niedrigen Anteil an oligomeren und polymeren Ricinolsäurederivaten auf.

## Beschreibung

Die vorliegende Erfindung betrifft ein Verfahren zur Herstellung von Zinkricinoleat unter Verwendung von Enzymen sowie die Verwender des so hergestellten Zinkrizinoleats.

Zinkricinoleat wird in vielen Anwendungen, z. B. in Wasch- und Reinigungsmitteln, aber insbesondere bei kosmetischen Zubereitungen als geruchsabsorbierendes Mittel eingesetzt. So ist beispielsweise aus der DE 40 14 055 A1 bekannt, Zinkricinoleat in Kombination mit ethoxylierten Fettalkoholen in Mitteln mit deodorierender Wirkung einzusetzen. Aus der WO 99/00090 sind Inkontinenzprodukte bekannt, welche luftdurchlässige Wände aufweisen, die geruchsabsorbierende Substanzen wie z. B. Zinkricinoleat enthalten können. Zur Herstellung derartiger Metallseifen geht man üblicherweise von Ölen aus, die direkt mit Metalloxiden oder -Salzen, wie beispielen Zinkoxid, Kalziumoxid oder Kalziumcarbonat umgesetzt werden. Von Nachteil ist jedoch, dass diese Reaktion hohe Umsetzungszeiten und Temperaturen benötigen, was dazu führen kann, dass die resultierenden Salze starke Verfärbungen und unerwünschte Anteile an Oligomeren und Polymeren aufweisen können. Häufig werden für derartige Reaktionen auch Schwermetallaktivatoren benötigt, die in der Mischung verbleiben und bei Anwendung speziell in der Kosmetik unerwünscht sind. Aus der DE 102 05 297 A1 ist nun ein Verfahren bekannt, bei dem Ricinusöl in einem ersten Schritt in ein ricinolsäurereiches Fettsäuregemisch und Glycerin aufgespalten wird, anschließend in einem zweiten Schritt das Glycerin abgetrennt wird und dann die verbleibende ricinolsäurereiche Fettsäuremischung mit einer Zinkverbindung zum gewünschten Endprodukt neutralisiert wird. Nachteilig bei der in der DE 102 05 297 A1 beschriebenen Vorgehensweise ist allerdings, dass mehrere voneinander getrennte Verfahrensschritte notwendig sind um das gewünschte Zinkricinoleat zu erhalten. Die Aufgabe der vorliegenden Erfindung bestand nun darin, ein gegenüber dem Stand der Technik verbessertes Herstellungsverfahren zu entwickeln.

Ein erster Gegenstand der vorliegenden Erfindung betrifft ein einstufiges Verfahren zur Herstellung von Zinkricinoleat, wobei man eine Mischung aus Wasser und Ricinusöl herstellt, diese mit einer Zinkverbindung vermischt und anschließend zusammen mit einem Enzym, welches geeignet ist, Ricinusöl in Glycerin und Ricinolsäure zu trennen, bei Temperaturen zwischen 10 und 70° C zur Reaktion bringt und nach Beendigung der Reaktion das Wasser abtrennt. Bei dieser Reaktionsführung wird zunächst durch das Enzym das Ricinusöl in Glycerin und Ricinolsäure getrennt und die freiwerdende Ricinolsäure wird unverzüglich mit der Zinkverbindung zum Zinkricinoleat umgesetzt. Das Zinkricinoleat liegt dann in wässriger Lösung vor. Da die freigesetzte Ricinolsäure unverzüglich mit dem Zinksalz zum Zinkricinoleat und Wasser reagiert, erfolgt keine Reaktion der Ricinolsäure zu Laktonen oder Oligo- bzw. Polymeren, wie sie aus dem Stand der Technik bekannt sind. Das so gebildete Zinkricinoleat hat daher einen hohen Gehalt an Aktivstoffen, ist farblos und aufgrund der einstufigen Verfahrensführung kostengünstiger als die bereits beschriebenen mehrstufigen Herstellungsverfahren.

Ricinusöl ist ein fast farbloses bis schwach gelbes, viskoses, brennbares und unverdauliches Öl mit sehr schwachem charakteristischen Geruch und unangenehmen Geschmack, das an der Luft verdickt, ohne jedoch in dünnen Filmen zu erstarren (d.h. kein "trocknendes"Öl). Seine Dichte beträgt 0,961-0,963 g/cm³ und der Schmelzpunkt -10°C bis -18°C, die Verseifungszahl wird mit 176-190, die Iodzahl mit 82-90 angegeben (Quelle Römpp Online, Version 2.11). Ricinusöl besteht zu 80-85% aus dem Glycerid der Rizinolsäure, daneben aus Glyceriden der Ölsäure (7%), Linolsäure (3%), Palmitinsäure (2%) u. Stearinsäure (1%). Die Rizinolsäure [(*R*)-12-Hydroxy-(*Z*)-Octadec-9-ensäure] hat die folgende Struktur:

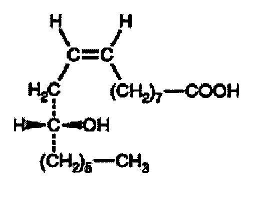

Im erfindungsgemäßen Verfahren wird als Zinkverbindung vorzugsweise basisches Zinkoxid (ZnO) verwendet. Dabei ist es vorteilhaft, dass pro Mol eingesetztem Ricinusöl zwischen 1,4 bis 3 Mol des Zinkoxids und vorzugsweise 1,45 bis 2 Mol und ganz besonders bevorzugt 1,5 bis 1,7 Mol der Zinkverbindung zum Einsatz kommen.

Die Reaktionstemperatur kann im Bereich von 10 bis 70° C liegen, wobei der Temperaturbereich von 15 bis 45° C und insbesondere der Bereich von 20 bis 35° C besonders bevorzugt ist. Ganz besonders vorteilhaft ist ein Temperaturbereich von 21 bis 30 ° C.

Da die eingesetzten Zinkverbindungen im Allgemeinen basisch sind, verläuft die Reaktion vorzugsweise im alkalischen pH-Wert, d. h. bei pH-Werten von größer 7 bis 14, vorzugsweise 9 bis 14, insbesondere zwischen 9 und 12 und insbesondere bei einem pH zwischen 9 und 11. Ein pH-Wert von größer/gleich 10 bis 12 oder bis 14 ist ein weiterer besonders bevorzugter Bereich. Die Reaktionsdauer ist dabei natürlich abhängig von den jeweils gewählten Reaktionsbedingungen, insbesondere - neben den Mengen, die zur Reaktion angesetzt werden - auch von den verwendeten Enzymen und Temperaturen und liegt üblicherweise zwischen 4 und 24 Stunden.

Wesentlich bei der oben skizzierten Reaktionsführung ist die Anwesenheit von Enzymen, die geeignet sind, das Ricinusöl, also ein Triglycerid der Ricinolsäure in Glycerin und die Fettsäure zu spalten. Vorzugsweise werden im erfindungsgemäßen Verfahren Lipasen eingesetzt und insbesondere solche aus der Gruppe der Thermomyces Lipasen. Ganz besonders bevorzugt sind weiterhin die Lipasen aus der Gruppe Thermomyces, Rhizomucor, Candida, insbesondere Candida cylindracea bzw. Candida rugosa, Fusarium, Geotrichum, Mucor und Rhizopus. Der Anteil der Enzyme beträgt bevorzugt 0,001 bis 1,5 Gew.-%, insbesondere 0,005 bis 1,2 Gew.-% und besonders bevorzugt 0,01 bis 1,5 Gew.-%, bezogen auf die Menge an eingesetztem Ricinusöl. Die Enzyme können in Form handelsüblicher Zubereitungen, die ggf. das Enzym in verdünnter Form enthalten, verwendet werden.

Das erfindungsgemäße Verfahren kann auch so ausgestaltet werden, dass zusätzlich zu den Inhaltsstoffen Wasser, Ricinusöl, Enzym und Zinkverbindung noch zusätzlich ein fester Träger mit zur Reaktion gebracht wird, wobei dieser vorzugsweise ausgewählt ist aus der Gruppe der Polysaccharide und / oder der Polypeptide und hier besonders der Stärken. Auf diese Weise entstehen feste Zinkricinoleatstärkepartikel, die z.B. durch Filtration aus der Reaktionslösung entfernt und anschließend nach Trocknung weiterverarbeitet werden können.

Nach Abschluss der Reaktion kann das Zinkricinoleat vom Wasser durch alle dem Fachmann bekannten Verfahren zur Fest-Flüssigseparationen getrennt werden und das so erhaltene feste Zinkricinoleat kann ggf. weiter über Trocknung und Mahlen konfektioniert werden. Beispiele für solche Separationsverfahren sind Dekantieren oder Zentrifugieren. Eine Alternative Vorgehensweise ist, dass man das Reaktionsgemisch nach Ablauf der Salzbildung zunächst auf 80 °C erwärmt, so dass das Zinkricinoleat in den flüssigen Zustand überführt wird, und dann eine Flüssig-flüssig-Trennung in dem Fachmann bekannter Art und Weise durchführt.

In dem erhaltenen Zinkricinoleat können noch erste an Glycerin enthalten sein, diese können beispielsweise durch Aufschmelzen und anschließendem Abkühlen, bei dem sich unter der Zinkricinoleatphase eine wässrige Glycerinwasserphase bildet, die beispielsweise durch dekantieren getrennt werden kann. Auf diese Weise ist es möglich, ein Zinkricinoleat mit Gehalt bis zu 99 % und größer zu erhalten. Es ist natürlich auch möglich, das Zinkricinoleat durch Zentrifugation vom Glycerin zu trennen. In dem so erhaltenen nach dem erfindungsgemäßen Verfahren hergestellten Zinkricinoleat können sich auch noch Enzymreste befinden, die beispielsweise durch kurzfristiges Erwärmen auf 80° C und höher deaktiviert werden können. Die genaue Temperatur, bei der eine Deaktivierung eintritt, hängt vom jeweils verwendeten Enzym ab, kann aber vom Fachmann leicht ermittelt werden.

In einer weiteren bevorzugten Ausführungsform wird das oben beschriebene Verfahren zur Herstellung von Zinkricinoleat angewendet und das daraus gewonnene Zinkricinoleat in Wasch- und Reinigungsmitteln oder kosmetischen Mitteln als Hilfsstoff und vorzugsweise als Geruchsabsorber verwendet. Eine weitere erfindungsgemäße Ausführungsform betrifft die Einarbeitung von Duftstoffen in Zinkricinoleat. Dazu wird das oben beschriebene Verfahren dahingehend erweitert, dass zum Reaktionsansatz bestehend aus Wasser, Zinkoxid bzw. Zinkverbindungen und dem Ricinusöl zusätzlich noch ein Parfümöl hinzu gegeben wird. Nach Abschluss der Reaktion erhält man dann ein Zinkricinoleat, das bereits gewisse Anteile des Parfümöls enthält und sich besonders zur Herstellung kosmetischer Mittel eignet.

Ein letzter Gegenstand der vorliegenden Erfindung betrifft ein Zinkricinoleat, hergestellt nach dem oben beschriebenen Verfahren und dadurch gekennzeichnet, dass der Anteil an polymeren oder oligomeren Ricinolsäurederivaten kleiner ist als 1 Gew.-% und insbesondere kleiner als 0,5 Gew.-% ist, jeweils bezogen auf den Aktivsubstanzanteil an Zinkricinoleat.

### Beispiele

### Beispiel 1: Enzymscreening zur Synthese von Zinkrizinoleat aus Ricinusöl

In 14 verschließbaren Gefäßen wurden jeweils 10 g Ricinusöl, 1,35 g Zinkoxid und 50 g Wasser eingewogen. Zu den Ansätzen wurden verschiedene Enzyme nach Tabelle 1 zudosiert. Die Ansätze wurden für 24 h auf einem Schüttelinkubator bei 30 °C inkubiert. Nach Beendigung der Reaktion wurde eine Probe des gebildeten Zinkrizinoleats entnommen und über Zentrifugation wird die Wasserphase abgetrennt. Die Proben wurden GC-chromatographisch auf den Gehalt an Zinkseifen sowie Glyceriden hin analysiert. Dazu wurden jeweils 20 mg der Zinkrizinoleat-Proben mit 1 ml BSTFA / MSTFA [6:1] Gemisch 30 min bei 80 °C inkubiert und anschließend auf einer DB5 HT Säule analysiert. Die Auswertung erfolgte über die Peakfläche. Zinkseifen wurden in Form der freien Säuren analysiert. Der Gehalt an Ricinolsäure im eingesetzten Ricinusöl und entsprechend in den gebildeten Zinkseifen beträgt dabei etwa 88 %. Die Zinkseifen werden im Folgenden vereinfacht Zinkrizinoleat genannt. Die Ergebnisse der Analysen finden sich in der Tabelle 2.

**Tabelle 1**

| **Ansatz** | **Enzym (Gew.-% auf Ricinusöl)** | **Hersteller** | **Organismus** |
|---|---|---|---|
| 1 | 1 % Lipolase | Novozymes | Thermomyces lanugenosus |
| 2 | 1 % Novozym 388 | Novozymes | Rhizomucor miehei |
| 3 | 1 % Novozym 525 | Novozymes | Candida antarctica B |
| 4 | 1 % Novozym 868 | Novozymes | Candida antarctica A |
| 5 | 1 % Lipase A | Amano | Aspergillus niger |
| 6 | 1 % Lipomod 34 | Biocatalysts | Candida cylindracea |
| 7 | 1 % Lipopan | Novozymes | Fusarium oxysporum |
| 8 | 0,5 % Lipase GC | Amano | Geotrichum candidum |
| 9 | 1 % Lipase M | Amano | Mucor javanicus |
| 10 | 1 % Lipase R | Amano | Penicilium roquefortii |
| 11 | 1 % Lipase L115P | Biocatalysts | Porcine Pancreas |
| 12 | 1 % Lipase PS | Amano | Pseudomonas sp. |
| 13 | 1 % Lipomod 36 | Biocatalysts | Rhizopus javanicus |
| 14 | 1 % Lipase F-AP 15 | Amano | Rhizopus oryzae |

**Tabelle 2**

| **Ansatz** | **Zinkrizinoleat** | **Monoglycerid** | **Diglycerid** | **Triglycerid** |
|---|---|---|---|---|
| 1 | 78,1% | 7,8% | 14,1% | 0% |
| 2 | 63,8% | 28,8% | 7,4% | 0% |
| 3 | 15,1% | 0,5% | 5,0% | 79,4% |
| 4 | 6,5% | 0% | 10,5% | 83,0% |
| 5 | 6,8% | 0% | 7,2% | 86,0% |
| 6 | 88,0% | 0% | 12,0% | 0% |
| 7 | 58,7 % | 33,3% | 8% | 0 % |
| 8 | 93,2 % | 0 % | 6,8% | 0 % |
| 9 | 60,4 % | 27,5 % | 12,1 % | 0 % |
| 10 | 37,5 % | 13,8 % | 47,6 % | 1,3 % |
| 11 | 18,2% | 0% | 0% | 81,8% |
| 12 | 5,6 % | 0 % | 8,3 % | 86,1 % |
| 13 | 92,1 % | 0 % | 2,0 % | 5,9 % |
| 14 | 90,4 % | 0% | 4,4 % | 9,6% |

Fazit: Alle getesteten Lipasen zeigen eine hydrolytische Reaktion bei Einsatz von Ricinusöl unter alkalischen Bedingungen durch Anwesenheit von ZnO im Reaktionsansatz. Besonders geeignet für eine einstufige Hydrolyse gekoppelt mit Zinkseifenbildung sind aus obigem Screening Lipasen / Esterasen bzw. Phospholipasen aus Thermomyces, Rhizomucor, Candida, insbesondere Candida cylindracea bzw. Candida rugosa, Fusarium, Geotrichum, Mucor und Rhizopus.

### Beispiel 2: Bildung von Zinkrizinoleat-Oligomeren in Abhängigkeit der Reaktionszeit

In eine Flasche wurden 10 g Ricinusöl, 50 g Wasser, 1,42 g Zinkoxid und 200 µl Lipozym TL 100 L (Novozymes) eingewogen. Der Ansatz wurde unter Rühren mit einem Magnetrührer verschlossen und bei Raumtemperatur inkubiert. Nach 6 h, 48 h und 72 h Reaktionszeit wurden Proben entnommen und GC-chromatographisch untersucht auf ihren Gehalt an nicht umgesetzten Ricinusöl-Glyceriden sowie gebildeten Rizinolsäure-Oligomeren. Dazu wurden 20 mg der Zinkrizinoleatproben mit 1 ml BSTFA / MSTFA [6:1] Gemisch 30 min bei 80 °C inkubiert und anschließend auf einer DB5 HT Säule analysiert. Die Auswertung erfolgt über die Peakfläche (siehe Tabelle 3). Zinkrizinoleat wird in Form der freien Ricinolsäure analysiert.

**Tabelle 3**

| **Reaktionszeit** | **Zinkrizinoleat** | **Glycerin** | **Glyceride** | **Oligomere** |
|---|---|---|---|---|
| | (Area %) | (Area %) | (Area %) | (Area %) |
| 5 h | 80,5% | 6,5% | 13,0% | < 0,5% |
| 24 h | 93,0% | 7,0% | <1,0% | <0,5% |
| 48 h | 92,2% | 7,8% | <1,0% | <0,5% |
| 72 h | 89,9% | 10,1% | <1,0% | <0,5% |

Der Gehalt an Glycerin variiert in Abhängigkeit der Menge an eingeschlossenem Wasser in der Produktprobe. Während der Reaktion bei Raumtemperatur wurden keine signifikanten Mengen an Rizinolsäure-Oligomeren gebildet. Gebildetes Zinkrizinoleat wird bei den niedrigen Reaktionstemperaturen nicht in Rizinolsäure-Oligomere umgewandelt.

### Beispiel 3: Synthese von Zinkrizinoleat in Abhängigkeit der ZnO-Konzentration

In 6 verschließbaren Gefäßen wurden jeweils 10 g Ricinusöl, 50 g Wasser und Zinkoxid in unterschiedlichen Anteilen (siehe Tabelle 4, unten) eingewogen. Zu den Ansätzen wurden jeweils 100 µl Lipozym TL 100 L (Novozymes) pipettiert. Die Ansätze wurden für 48 h auf einem Schüttelinkubator bei 30 °C inkubiert. Nach 5 h wurde aus allen Ansätzen und nach 48 h wurden aus den Ansätzen 1 und 6 eine Probe des gebildeten Zinkrizinoleats entnommen und über Zentrifugation wird die Wasserphase abgetrennt. Die Proben wurden, wie in Beispiel 1 beschreiben, GC-chromatographisch auf den Gehalt an restlichen nicht umgesetzten Glyceriden analysiert.

**Tabelle 4**

| **Ansatz** | **Zinkoxid** | **Zinkoxid-Überschuss** | **Glyceride nach 5 h (Area %)** | **Glyceride nach 48 h (Area %)** |
|---|---|---|---|---|
| 1 | 1,29 g | 0 % | 32,7 % | 2,3 % |
| 2 | 1,35 % | 5 % | 45,9 % | |
| 3 | 1,42 g | 10 % | 48,6 % | |
| 4 | 1,55 g | 20 % | 43,8 % | |
| 5 | 1,68 g | 30 % | 50,6 % | |
| 6 | 1,93 g | 50 % | 40,0 % | < 1,0 % |

Fazit: Der Einfluss der ZnO-Konzentration auf die Reaktionsgeschwindigkeit sowie auf die maximale Produktbildung ist gering. Es wird ein hoher Umsatz von Ricinusöl sowohl bei einer stöchiometrischen Zugabe von ZnO als auch bei einem 1,5 fachen molaren Überschuss an ZnO erreicht.

### Beispiel 4: Synthese von Zinkrizinoleat unter Dosierung von Ricinusöl

In einem 6 l Reaktor werden 5 kg Wasser, 81 g ZnO und 15 g Lipolase vorgelegt. Unter Rühren werden 600 g Ricinusöl über einen Zeitraum von 6 h bei Raumtemperatur zudosiert. Nach 3 h und 5 h werden jeweils weitere 5 g Lipolase und 10 g ZnO in den Reaktor gegeben. Es bilden sich feine Zinkrizinoleatpartikel.
Ergebnis: Nach 24 h wird eine Probe des Zinkrizinoleats gaschromatographisch untersucht. Der Umsatz von Ricinusöl zu Zinkrizinoleat ist vollständig. Die Zinkrizinoleat-Suspension wird für insgesamt 72 h gerührt. Die feinen Partikel klumpen nicht zusammen. Nach 72 h wird eine Probe des Zinkrizinoleatpulvers bei Raumtemperatur getrocknet und mikroskopisch auf seine Teilchengröße untersucht. Die Teilchengröße des Zinkrizinoleats liegt zwischen 5 und 200 µm.

### Beispiel 5: Synthese von Zinkrizinoleat unter Dosierung von Ricinusöl in Anwesenheit eines Trägermaterials

In einem 6 l Reaktor werden 5 kg Wasser, 81 g ZnO, 60 g Stärke und 15 g Lipolase vorgelegt. Unter Rühren werden 600 g Ricinusöl über einen Zeitraum von 6 h bei Raumtemperatur zudosiert. Nach 3 h und 5 h werden jeweils weitere 5 g Lipolase und 10 g ZnO in den Reaktor gegeben. Es bilden sich feine Zinkrizinoleatpartikel.
Ergebnis: Nach 24 h wird eine Probe des Zinkrizinoleats gaschromatographisch untersucht. Der Umsatz von Ricinusöl zu Zinkrizinoleat ist vollständig. Die Zinkrizinoleat-Suspension wird für insgesamt 72 h gerührt. Die feinen Partikel klumpen nicht zusammen. Nach 72 h wird eine Probe des Zinkrizinoleatpulvers bei Raumtemperatur getrocknet und mikroskopisch auf seine Teilchengröße untersucht. Die Teilchengröße des Zinkrizinoleats liegt zwischen 5 und 200 µm. Im Gegensatz zu Beispiel 9 liegt der Anteil von Partikeln mit kleiner Größe höher.

### Beispiel 6: Einarbeitung von Duftstoff-Additiven in Zinkrizinoleat

In zwei verschließbaren Gefäßen wurden jeweils 10 g Ricinusöl, 50 g Wasser und 1,35 g Zinkoxid eingewogen. Zu Ansatz 1 werden 5 Gew.-% Orangenöl und zu Ansatz 2 werden 5 Gew.-% Phenylalkohol gegeben. Die Reaktionen wurden jeweils durch Zugabe von 200 µl Lipozym TL 100 L (Novozymes) gestartet. Die Ansätze werden für 24 h bei Raumtemperatur gerührt. Das gebildete Zinkrizinoleat wurde abfiltriert und ohne vorherige Trocknung gaschromatographisch auf den Anteil an gebundenem Duftstoff analysiert. Ergebnis: 80% des eingesetzten Orangenöls und 72 % des Phenylalkohols wurden im Zinkrizinoleat nachgewiesen.

## Patentansprüche

1. Einstufiges Verfahren zur Herstellung von Zinkricinoleat, wobei man Wasser und Ricinusöl in Anwesenheit einer Zinkverbindung bei Temperaturen von 10 bis 70 °C mit einem Enzym, welches geeignet ist Ricinusöl in Glycerin und Rizinolsäure zu Trennen, zur Reaktion bringt und nach Beendigung der Reaktion das Wasser abtrennt.

2. Verfahren nach Anspruch 1, **dadurch gekennzeichnet, dass** als Zinkverbindung ZnO verwendet wird.

3. Verfahren nach den Ansprüchen 1 bis 2, **dadurch gekennzeichnet, dass** der pH-Wert der wässerigen Phase während der Reaktion bei 9 bis 14, vorzugsweise bei 9 bis 12 und insbesondere bei 9 bis 11 beträgt.

4. Verfahren nach den Ansprüchen 1 bis 3, **dadurch gekennzeichnet, dass** die Reaktionstemperatur 15 °C bis 45 °C, vorzugsweise von 20 °C bis 35 ° und insbesondere von 21 °C bis 30 °C beträgt.

5. Verfahren nach den Ansprüchen 1 bis 4, **dadurch gekennzeichnet, dass** pro Mol Ricinusöl 1,4 bis 3 Mol der Zinkverbindung, vorzugsweise 1,45 bis 2 Mol der Zinkverbindung und insbesondere 1,5 bis 1,7 Mol der Zinkverbindung eingesetzt wird.

6. Verfahren nach den Ansprüchen 1 bis 5, **dadurch gekennzeichnet, dass** Wasser und Ricinusöl im Gewichtsverhältnis von 1 : 1 bis 10 : 1, und vorzugsweise im Gewichtsverhältnis von 2 :1 bis 5 : 1 eingesetzt wird.

7. Verfahren nach den Ansprüchen 1 bis 6, **dadurch gekennzeichnet, dass** das Enzym ausgewählt ist aus der Gruppe der Lipasen und vorzugsweise aus der Gruppe der Thermomyces Lipasen.

8. Verfahren nach den Ansprüchen 1 bis 7, aus der Gruppe Thermomyces, Rhizomucor, Candida, isb. cylindracea bzw, rugosa, Fusarium, Geotrichum, Mucor und Rhizopus.

9. Verfahren nach den Ansprüchen 1 bis 7, **dadurch gekennzeichnet, dass** während der Reaktion zusätzlich ein fester Träger anwesend ist, vorzugsweise ausgewählt aus der Gruppe der Polypeptide und/oder Polysaccharide und vorzugsweise der Stärken.

10. Verwendung von Zinkrizinoleat, hergestellt nach dem Verfahren gemäß den Ansprüchen 1 bis 8, zur Herstellung von Wasch- und Reinigungsmitteln, oder von Kosmetika.

11. Verwendung von Zinkrizinoleat, hergestellt nach dem Verfahren gemäß den Ansprüchen 1 bis 9, als Geruchsabsorber.

12. Zinkricinoleat, hergestellt nach dem Verfahren der Ansprüche 1 bis 8, **dadurch gekennzeichnet, dass** der Anteil an polymeren oder oligomeren Rizinolsäurederivaten kleiner als 1 Gew.-% und insbesondere kleiner als 0,5 Gew.-% bezogen auf den Aktivsubstanzanteil an Zinkrizinoleat beträgt.
